# EUROPEAN PATENT SPECIFICATION

(11) **EP 4 599 762 B1**
(45) Date of publication and mention of the grant of the patent: **09.12.2026**
(21) Application number: 24305208.1
(22) Date of filing: 08.02.2024
(51) Int. Cl.: A61B 5/103, B29C 64/386, B33Y 80/00, G06F 30/15, B62J 1/00, B62J 1/26

(54) **METHOD OF MANUFACTURING A CUSTOMIZED SADDLE CUSHION**
VERFAHREN ZUR HERSTELLUNG EINES INDIVIDUELL ANGEPASSTEN SATTELKISSENS
PROCÉDÉ DE FABRICATION D'UN COUSSIN DE SELLE PERSONNALISÉ

(43) Date of publication of application: 13.08.2025
(73) Proprietor: Relance SAS, 78000 Versailles (FR)
(72) Inventor: BOIVIN, Jean-Rémi, 78280 GUYANCOURT (FR)
(74) Representative: Plasseraud IP

(56) References cited:
- WO-A1-2020/090798
- WO-A2-2022/185262
- DE-U1- 202010 008 146
- FR-B1- 3 141 674
- US-A1- 2021 294 936
- US-B2- 10 722 166

## Description

### Technical Field

The invention pertains to the field of saddles and more specifically to saddle cushions, notably for bicycles.

### Background Art

Bicycle saddles can become extremely uncomfortable after long hours riding. To alleviate pressure points on the ischium and pubis areas, saddles may have sections of different thickness or foam rigidities.

WO 2022/185262 A2 discloses a method of manufacturing a customized saddle.

To improve comfort, some have proposed to mold the saddle anatomically onto the rider before manufacturing it. This solution only takes into account the shape of the buttock area of the rider.

The above solutions do not provide a custom fit solution, especially in dynamic situations, such as when riding the bicycle.

### Summary of the invention

It is proposed a method of manufacturing a customized saddle cushion, such as a bicycle saddle cushion, the method comprising: applying a molding paste onto a saddle shell to form a probe saddle, the molding paste being deformable under a weight of the rider; mounting the probe saddle onto a bicycle and letting the molding paste of the probe saddle deform under the weight and motion of a rider riding the bicycle during a riding session, the riding session including at least one active riding position; after the deformed molding paste has reached a geometrically stable state, positioning a plurality of pressure sensors onto the probe saddle and recording pressures from the plurality of pressure sensors during another performance by the rider of the riding session; obtaining a 2-D pressure map on the geometrically stable deformed molding paste by selecting for each pressure sensor the highest recorded pressure during the riding session; obtaining a 3-D saddle cushion model of the geometrically stable deformed molding paste; and associating the 3-D saddle cushion model with the 2-D pressure map to obtain a 3-D saddle cushion pressure mesh to be used as a reference for 3-D printing the customized saddle cushion, a density of the 3-D saddle cushion pressure mesh being correlated to the 2-D pressure map.

The following features, can be optionally implemented, separately or in combination one with the others:
- the method further comprises 3-D printing the customized saddle cushion.
- the molding paste includes a plurality of layers of pastes having different rigidities.
- the layers of pastes are disposed on top of one another by level of rigidity starting from a paste having the highest rigidity disposed proximate to the saddle shell.
- the molding paste is at least one of a mineral paste, thermoactivated, and reusable.
- the molding paste is plastically deformable at a temperature range ranging from ambient temperature to body temperature.
- positioning the plurality of pressure sensors onto the probe saddle includes positioning onto the probe saddle a mat comprising the plurality of pressure sensors.
- obtaining the 3-D saddle cushion model includes obtaining the 3-D saddle cushion model by at least one of scanning, radiography, and photography.
- associating the 3-D saddle cushion model with the 2-D pressure map includes: forming a 3-D pressure map of the 2-D pressure map and associating it with the 3-D saddle cushion model.
- forming the 3-D pressure map of the 2-D pressure map includes applying a constant additional one dimension to the 2-D map.
- the riding session includes two or more active riding positions where the rider is pedaling, and the two or more active riding positions being at least two or more of: rider arms up straight back, rider arms down straight back, rider with hands on handles of the bicycle and straight back, rider with hands on handles while leaning forward toward the handles.
- the molding paste can be deformed, and optionally hardened back and forth, a plurality of times.
- the 3-D saddle cushion pressure mesh is made of open cells.
- the method further comprises assembling the 3-D printed customized saddle cushion to the saddle shell to form a customized saddle and mounting the customized saddle onto the bicycle; positioning the plurality of pressure sensors onto the customized saddle; recording pressure from the plurality of pressure sensors during yet another performance by the rider of the riding session; obtaining a 2-D pressure map of the customized saddle cushion by selecting for each pressure sensor the highest recorded pressure during the riding session; and comparing the 2-D pressure map of the customized saddle with the 2-D pressure map of the probe saddle, if the 2-D pressure map of the customized saddle coincides with the 2-D pressure map of the probe saddle, determining that the customized saddle cushion is adequate, else repeating all the steps of the method until the customized saddle cushion is adequate.
- the method further comprises mounting the customized saddle cushion to the saddle shell, and optionally covering it with a finishing layer, such as a leather layer.

### Brief Description of Drawings

Other features, details and advantages will be shown in the following detailed description and on the figures, on which:
**Fig. 1**

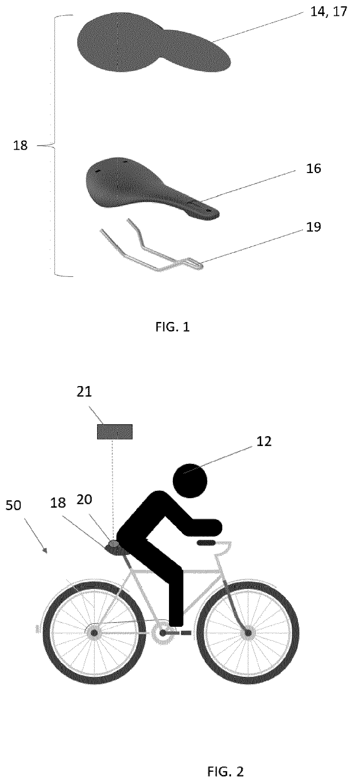

   [Fig. 1] is an exploded view of a probe saddle according to an embodiment;
**Fig. 2**
   [Fig. 2] is a schematic of the probe saddle mounted onto a bicycle according to an embodiment;
**Fig. 3**

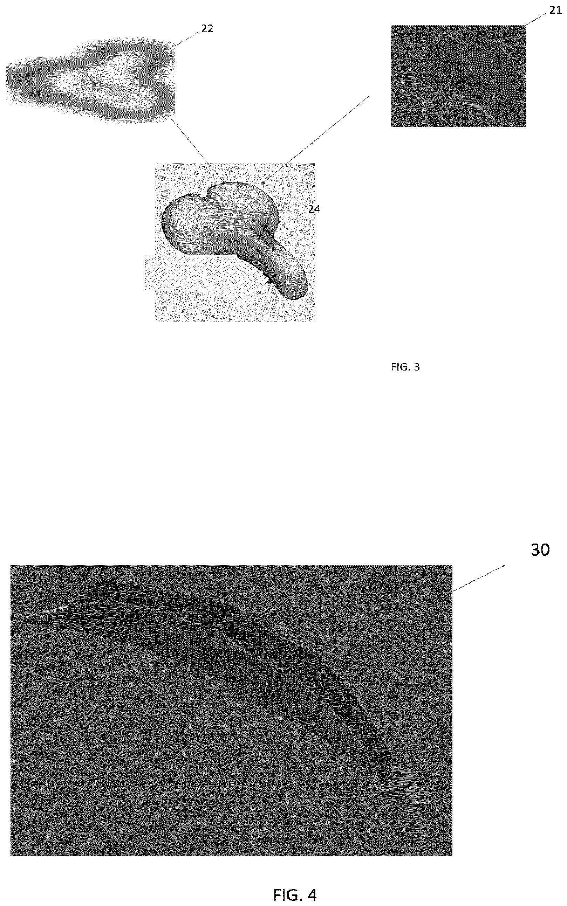

   [Fig. 3] is an example of 2-D pressure map and 3-D modeling of a customized saddle cushion according to an embodiment; and
**Fig. 4**
   [Fig. 4] is an example of a cross section of 3-D printed customized saddle cushion according to an embodiment.

### Description of Embodiments

A **method 10** of manufacturing a saddle cushion customized to a **rider 12,** herein **customized saddle cushion 30,** will be described. Although the method 10 is described herein in the context of a bicycle saddle, it is understood that the method could be applied to other types of saddle cushions, such as motorbikes. The method 10 allows to manufacture a true customized saddle cushion, which takes into account not only the anatomy of the rider, but also the various positions he/she has while riding/pedaling the bicycle. To do so, an anatomically correct model of the saddle cushion is determined and pressures on the saddle while riding/pedaling are being recorded.

The method 10 starts with the obtention of a **probe saddle 18.** In a **first step 10-1,** a **molding paste 14** is applied onto a **saddle shell 16** to form the probe saddle 18. The molding paste 14 is a blank for the rider to imprint its buttock / crotch area while riding the bicycle. The molding paste 14 does not have a specific shape, and may in one embodiment have roughly the shape of a **saddle cushion 17** as illustrated in Figure 1. In another example, the molding paste 14 may not have initially a specific paste, and the shape of the molding paste 14 may be adjusted to have the shape of the saddle cushion 17 later on.

The probe saddle 18 will be, in a later step, deformed under a weight and motion of the rider 12 riding the bicycle allowing to take into account the anatomy and riding positions of the rider 12. To do so, the molding paste 14 is a deformable material chosen to deform under the weight of the rider 12. Once deformed, the molding paste 14 is geometrically stable in the normal conditions of operation (e.g. ambient temperature). The molding paste 14 is thus chosen to deform when the rider 12 is on it and to not deform when the rider 12 isn't on it. Having a plastically deformable material allows to later 3D model its shape which has been deformed by the rider seating and moving on it. The ability of the molding paste 14 to deform may be chosen in function of the weight and/or body type of the rider 12. In one example, the molding paste 14 is made of a plastically deformable material, that may or may not be hardened after deformation.

In one example, the molding paste 14 is a nonhardening modeling clay made from clay mixed with oil or wax. In another example, the molding paste 14 is a mineral clay that may or may not be hardened. In one example, the molding paste 14 may be hardened after deformation in order to keep the deformed state. Hardening may occur by different processes depending on the type of molding paste 14 used. For example, hardening may occur by letting the molding paste 14 untouched at ambient temperature during a sufficient time, for example 4 to 5 hours. If the molding paste 14 is a breakable foam (for example the cells of the foam brake irreparably under a constraint), the hardening is instantaneous when the rider 12 is removed from the probe saddle. Hardening could also occur by putting the molding paste in a freezer. In view of the different non limitative examples above, it is understood that hardening thus does not have to be an active step.

In one example, the molding paste 14 is plastically deformable at a temperature range of the conditions in which the method 10 is performed, for example ranging from ambient temperature to body temperature. The molding paste 14 may be thermoactivated, for example it is rather hard at ambient temperature, but plastically deformable at temperature corresponding to body temperature. The molding paste 14 may be reusable. In that case, after the method 10 is completed, the molding paste can be once again deformed in order to become again the blank of step 10-1. The same molding paste can be thus deformed (and optionally hardened back and forth) a plurality of times in order to reduce waste to the environment. The reusability of the molding paste 14 may be achieved, for example, by exposing the paste 14 to heat so to reshape it into a blank.

Different thicknesses and rigidities of the molding paste 14 used as a blank onto the saddle shell 16 can be chosen to accommodate the anatomy of the rider 12. For example, a rider 12 with low body fat, may have pointy ischium, and as a result may deform the molding paste 14 is a greater manner than a rider with more body fat. A higher rigidity of the molding paste 14 is chosen in that case. The molding paste 14, in one example, has a rigidity comprised between 30 and 70 Shores. For example, for a heavier rider the molding paste 14 formed with the molding paste 14 may be thicker.

The molding paste 14 may be composed of more than one layer. For example, molding paste 14 may be made of two or more layers of paste having different rigidities. In one example, if the molding paste 14 has two or more layers, the layer of the highest rigidity is chosen to be the one proximate to the saddle shell 16, and the layer of the lowest rigidity is chosen to be the one distal to the saddle shell 16. The layers in between may be arranged by decreasing rigidities from the layer of the highest rigidity to the layer of lowest rigidity. In one example, the layers are separated by a thin film, for example a plastic film such as polypropylene. The films may allow the layers to not blend into each other when they deform, and the reuse each layer after the method 10 is performed.

The saddle shell 16 may be obtained from an existing saddle, by stripping any soft material from it in order to reuse the saddle shell 16, or may be a new saddle shell 16. The saddle shell 16 is made of a rigid non deformable material, for example plastic or metal or composite.

The probe saddle 18 may also include **rails 19.**

To form the probe saddle 18, the molding paste 14 may be simply laid over the saddle shell 16. In other embodiment, an attachment system is used.

Once the probe saddle 18 is formed, the method 10 goes to a **second step 10-2,** where the probe saddle 18 is mounted onto a **bicycle 50** for performing a riding session as shown in Figure 2. The rider 12 goes onto the bicycle 50 with the probe saddle 18 thereon and performs a riding session. The riding session includes one or more active riding positions. By active riding position it is understood a position of the rider on the bicycle 50 while the rider 12 is actively pedaling. Examples of riding positions include non-exhaustively: rider arms up straight back, rider arms down straight back, rider with hands on handles of the bicycle and straight back, rider with hands on handles while leaning forward toward the handles. When performing step 10-2, the bicycle 50 may be a static bicycle.

As a result of the weight and motion of the rider riding the bicycle 50 during a riding session, the molding paste 14 deforms plastically. This means that for any position of the rider 12 on the bicycle 50 there is a deformation of the molding paste 14, and that the molding paste 14 does not return to a different shape between the various riding positions. The molding paste 14 behaves in a way that it records in a cumulative manner all the deformations induced by the rider's active riding positions.

After the riding session, the rider 12 is removed from the probe saddle 18. If the molding paste 14 doesn't require hardening (for example, the molding paste is already geometrically stable) it is left as it, else the molding paste 14 is hardened to be geometrically stable. For the hardening to happen the molding paste 14 may not have to be removed from the saddle shell 16. As explained before, the hardening may be instantaneous, or may need time to happened. Should the hardening require the molding paste 14 to be for example baked, the molding paste 14 may be removed from the saddle shell 16. Once the molding paste 14 is geometrically stable after its deformation, the method 10 goes to a **step 10-3**, where a plurality of **pressure sensors 20** are disposed onto the probe saddle 18, as shown in Figure 2. The pressure sensors 20 may for example be embedded in a mat such that the mat is simply disposed over the probe saddle 18. Because the molding paste 14 is geometrically stable, the positioning of the pressure sensors 20 does not deform the molding paste 14 further.

The pressure sensors 20 are connected to a **control unit 21** which allows to record the pressures from the plurality of pressure sensors 20. At step 10-3, the rider 12 thus performs the same riding session as at step 10-2 and the pressures induced by its moving body onto the saddle probe 18 are recorded. The riding session includes the same positions in the same order as the riding session of step 10-2 or includes the same positions but in a different order as the riding session of step 10-2. The pressure recorded may be stored in a storage device (non illustrated).

At **step 10-4,** when the riding session is completed and the various pressure recorded, a **2-D pressure map 22** (shown in Figure 3) is obtained by selecting for each pressure sensor 20 the highest recorded pressure during the riding session.

At **step 10-5,** the deformed molding paste 14 obtained at the end of step 10-2 (i.e. geometrically stable deformed molding paste) is modeled into a **3-D saddle cushion model 21** (shown in Figure 3). The step 10-5 may be performed right after the deformed molding paste 14 is geometrically stable at the end of step 10-2, or after steep 10-4. The 3-D saddle cushion model 21 of the geometrically stable deformed molding paste molding paste 14 may be obtained using one or more of scanning, radiography, and photography. The 3-D saddle cushion model 21 is stored in the form of a computer readable file. The 3-D saddle cushion model 21 allows to have a virtual version of the shape of the deformed cushion (i.e. molding paste 14) of the probe saddle 18.

At **step 10-6,** the 3-D saddle cushion model 21 is associated with the 2-D pressure map 22 to obtain a **3-D saddle cushion pressure mesh 24** (shown in Figure 3). The 3-D saddle cushion pressure mesh 24 will be used as a reference for 3-D printing a **customized saddle cushion 30** (shown in Figure 4). To allow the association of the 3-D saddle cushion model 21 with the 2-D pressure map, the 2-D pressure map may be first converted into a 3-D pressure map. Converting the 2-D into a 3-D map may be obtained by assigning a third dimension of constant value to the 2-D map.

To enable the association of the 3-D saddle cushion model 21 with the 3-D map, the 3-D saddle cushion model 21 is first converted into a mesh. This involves breaking down the object's surfaces into small geometric elements (usually triangles) that collectively represent the overall shape. The result of this combination is the 3-D saddle cushion pressure mesh 24, where a density of the 3-D saddle cushion pressure mesh 24 is correlated to the 2-D pressure map. The mesh may have open cells. The smaller the cells, the denser the mesh is. In 3-D printing, the denser the mesh, the higher the rigidity of the finish product. Thus, zones of the 3-D saddle cushion pressure mesh 24 with a high density of cells correspond to zones of the 2-D pressure map having recorded a high pressure. By adjusting the rigidity of the customized saddle cushion 30 (via the density of the mesh), pressures on the customized saddle are uniformized. High pressures areas of the customized saddle cushion 30 are thus made of a more rigid (via a higher density of cells) material. An external surface of the 3-D saddle cushion pressure mesh 24 may be plain (i.e. without open cells).

At **step 10-7,** the customized saddle cushion 30 may be 3-D printed using the 3-D saddle cushion pressure mesh 24 which includes rigidity values correlated to the 2-D pressure map. The material used to 3-D print the customized saddle cushion 30 may be a thermoplastic monomer. The thermoplastic monomer may for example have a rigidity of 92A to 95A Shores. A fully customized saddle may thus be obtained by mounting the customized saddle cushion 30 to the saddle shell 16 and optionally covering it with a finishing layer, such as a leather layer.

At **step 10-8,** a further optional validation sequence is performed to ensure that the customized saddle cushion 30 takes correctly into account the 2-D pressure map. To do so, the 3-D printed customized saddle cushion 30 is assembled to the saddle shell 16 to form the customized saddle. The plurality of sensors 20 are positioned, preferably in the exact same location as in step 10-3, onto the customized saddle cushion 30. The rider 12 is requested to perform the riding session previously performed in step 10-2 or step 10-3, and the pressure from the plurality of pressure sensors 20 is recorded. A 2-D pressure map of the customized saddle cushion 30 is obtained by selecting for each pressure sensor the highest recorded pressure during the riding session. The 2-D pressure map of the customized saddle is compared with the 2-D pressure map of the probe saddle 18. If the map of the pressures of the customized saddle coincides with the 2-D pressure map of the probe saddle 18, then the customized saddle cushion 30 is considered adequate. If not, manufacturing of the customized saddle cushion 30 is repeated starting at step 10-1.

## Claims

1. **Method (10)** of manufacturing a **customized saddle cushion (30),** such as a bicycle saddle cushion, the method comprising:
a. Applying a **molding paste (14)** onto a **saddle shell (16)** to form a **probe saddle (18),** the molding paste being deformable under a weight of the rider;
b. Mounting the probe saddle onto a bicycle and letting the molding paste of the probe saddle deform under the weight and motion of a **rider (12)** riding the bicycle during a riding session, the riding session including at least one active riding position;
c. After the deformed molding paste has reached a geometrically stable state, positioning a plurality of **pressure sensors (20)** onto the probe saddle and recording pressures from the plurality of pressure sensors during another performance by the rider of the riding session;
d. Obtaining a **2-D pressure map (22)** on the geometrically stable deformed molding paste by selecting for each pressure sensor the highest recorded pressure during the riding session;
e. Obtaining a **3-D saddle cushion model (21)** of the geometrically stable deformed molding paste; and
f. Associating the 3-D saddle cushion model with the 2-D pressure map to obtain a **3-D saddle cushion pressure mesh (24)** to be used as a reference for 3-D printing the customized saddle cushion, a density of the 3-D saddle cushion pressure mesh being correlated to the 2-D pressure map.

2. Method according to the preceding claim, further comprising 3-D printing the customized saddle cushion.

3. Method according to the preceding claim, wherein the molding paste includes a plurality of layers of pastes having different rigidities.

4. Method according to the preceding claim, wherein the layers of pastes are disposed on top of one another by level of rigidity starting from a paste having the highest rigidity disposed proximate to the saddle shell.

5. Method according to any of the preceding claims, wherein the molding paste is at least one of a mineral paste, thermoactivated, and reusable.

6. Method according to any of the preceding claims, wherein the molding paste is plastically deformable at a temperature range ranging from ambient temperature to body temperature.

7. Method according to any of the preceding claims, wherein positioning the plurality of pressure sensors onto the probe saddle includes positioning onto the probe saddle a mat comprising the plurality of pressure sensors.

8. Method according to any of the preceding claims, wherein obtaining the 3-D saddle cushion model includes obtaining the 3-D saddle cushion model by at least one of scanning, radiography, and photography.

9. Method according to any of the preceding claims, wherein associating the 3-D saddle cushion model with the 2-D pressure map includes: forming a 3-D pressure map of the 2-D pressure map and associating it with the 3-D saddle cushion model.

10. Method according to any of the preceding claims, wherein forming the 3-D pressure map of the 2-D pressure map includes applying a constant additional one dimension to the 2-D map.

11. Method according to any of the preceding claims, wherein the riding session includes two or more active riding positions where the rider is pedaling, and the two or more active riding positions being at least two or more of: rider arms up straight back, rider arms down straight back, rider with hands on handles of the bicycle and straight back, rider with hands on handles while leaning forward toward the handles.

12. Method according to any of the preceding claims, wherein the molding paste can be deformed, and optionally hardened back and forth, a plurality of times.

13. Method according to any of the preceding claims, wherein the 3-D saddle cushion pressure mesh is made of open cells.

14. Method according to any of claims 2 to 13, further comprising:
a. Assembling the 3-D printed customized saddle cushion to the saddle shell to form a customized saddle and mounting the customized saddle onto the bicycle;
b. Positioning the plurality of pressure sensors onto the customized saddle;
c. Recording pressure from the plurality of pressure sensors during yet another performance by the rider of the riding session;
d. Obtaining a 2-D pressure map of the customized saddle cushion by selecting for each pressure sensor the highest recorded pressure during the riding session; and
e. Comparing the 2-D pressure map of the customized saddle with the 2-D pressure map of the probe saddle, if the 2-D pressure map of the customized saddle coincides with the 2-D pressure map of the probe saddle, determining that the customized saddle cushion is adequate, else repeating all the steps of the method until the customized saddle cushion is adequate.

15. Method according to any of claims 2 to 14, further comprising mounting the customized saddle cushion to the saddle shell, and optionally covering it with a finishing layer, such as a leather layer.

## Patentansprüche

1. Verfahren **(10)** zur Herstellung eines **maßgeschneiderten Sattelpolsters (30)**, wie beispielsweise eines Fahrradsattelpolsters, wobei das Verfahren umfasst:
a. Aufbringen einer **Modellierpaste (14)** auf eine **Sattelschale (16)**, um einen **Prüfsattel (18)** zu bilden, wobei die Modellierpaste unter einem Gewicht des Fahrers verformbar ist;
b. Montieren des Prüfsattels auf ein Fahrrad und Verformenlassen der Modellierpaste des Prüfsattels unter dem Gewicht und der Bewegung eines **Fahrers (12)**, der das Fahrrad während einer Fahrsitzung fährt, wobei die Fahrsitzung mindestens eine aktive Fahrposition beinhaltet;
c. nachdem die verformte Modellierpaste einen geometrisch stabilen Zustand erreicht hat, Positionieren einer Vielzahl von **Drucksensoren (20)** auf dem Prüfsattel und Aufzeichnen von Drücken von der Vielzahl von Drucksensoren während einer weiteren Durchführung der Fahrsitzung durch den Fahrer;
d. Erhalten einer **2D-Druckkarte (22)** auf der geometrisch stabilen verformten Modellierpaste durch Auswählen des höchsten aufgezeichneten Drucks während der Fahrsitzung für jeden Drucksensor;
e. Erhalten eines **3D-Sattelpolstermodells (21)** der geometrisch stabilen verformten Modellierpaste; und
f. Zuordnen des 3D-Sattelpolstermodells zu der 2D-Druckkarte, um ein **3D-Sattelpolster-Drucknetz (24)** zu erhalten, das als Referenz für das 3D-Drucken des maßgeschneiderten Sattelpolsters zu verwenden ist, wobei eine Dichte des 3D-Sattelpolster-Drucknetzes mit der 2D-Druckkarte korreliert ist.

2. Verfahren nach dem vorhergehenden Anspruch, ferner umfassend das 3D-Drucken des maßgeschneiderten Sattelpolsters.

3. Verfahren nach dem vorhergehenden Anspruch, wobei die Modellierpaste eine Vielzahl von Schichten aus Pasten mit unterschiedlichen Steifigkeiten beinhaltet.

4. Verfahren nach dem vorhergehenden Anspruch, wobei die Schichten aus Pasten nach Steifigkeitsniveau übereinander angeordnet werden, beginnend mit einer Paste mit der höchsten Steifigkeit, die nahe der Sattelschale angeordnet ist.

5. Verfahren nach einem der vorhergehenden Ansprüche, wobei die Modellierpaste mindestens eine aus einer Mineralpaste, thermoaktiviert und wiederverwendbar ist.

6. Verfahren nach einem der vorhergehenden Ansprüche, wobei die Modellierpaste in einem Temperaturbereich von Umgebungstemperatur bis Körpertemperatur plastisch verformbar ist.

7. Verfahren nach einem der vorhergehenden Ansprüche, wobei das Positionieren der Vielzahl von Drucksensoren auf dem Prüfsattel das Positionieren einer Matte, die die Vielzahl von Drucksensoren umfasst, auf dem Prüfsattel beinhaltet.

8. Verfahren nach einem der vorhergehenden Ansprüche, wobei das Erhalten des 3D-Sattelpolstermodells das Erhalten des 3D-Sattelpolstermodells durch mindestens eines aus Scannen, Radiographie und Fotografie beinhaltet.

9. Verfahren nach einem der vorhergehenden Ansprüche, wobei das Zuordnen des 3D-Sattelpolstermodells zu der 2D-Druckkarte beinhaltet: Bilden einer 3D-Druckkarte aus der 2D-Druckkarte und Zuordnen dieser zu dem 3D-Sattelpolstermodell.

10. Verfahren nach einem der vorhergehenden Ansprüche, wobei das Bilden der 3D-Druckkarte aus der 2D-Druckkarte das Anwenden einer konstanten zusätzlichen Dimension auf die 2D-Karte beinhaltet.

11. Verfahren nach einem der vorhergehenden Ansprüche, wobei die Fahrsitzung zwei oder mehr aktive Fahrpositionen beinhaltet, in denen der Fahrer pedaliert, und wobei die zwei oder mehr aktiven Fahrpositionen mindestens zwei oder mehr sind aus: Fahrer Arme oben mit geradem Rücken, Fahrer Arme unten mit geradem Rücken, Fahrer mit Händen an den Griffen des Fahrrads und geradem Rücken, Fahrer mit Händen an den Griffen, während er sich nach vorne zu den Griffen lehnt.

12. Verfahren nach einem der vorhergehenden Ansprüche, wobei die Modellierpaste eine Vielzahl von Malen hin und her verformt und optional gehärtet werden kann.

13. Verfahren nach einem der vorhergehenden Ansprüche, wobei das 3D-Sattelpolster-Drucknetz aus offenen Zellen besteht.

14. Verfahren nach einem der Ansprüche 2 bis 13, ferner umfassend:
a. Zusammenfügen des 3D-gedruckten maßgeschneiderten Sattelpolsters mit der Sattelschale, um einen maßgeschneiderten Sattel zu bilden, und Montieren des maßgeschneiderten Sattels auf das Fahrrad;
b. Positionieren der Vielzahl von Drucksensoren auf dem maßgeschneiderten Sattel;
c. Aufzeichnen des Drucks von der Vielzahl von Drucksensoren während noch einer weiteren Durchführung der Fahrsitzung durch den Fahrer;
d. Erhalten einer 2D-Druckkarte des maßgeschneiderten Sattelpolsters durch Auswählen des höchsten aufgezeichneten Drucks während der Fahrsitzung für jeden Drucksensor; und
e. Vergleichen der 2D-Druckkarte des maßgeschneiderten Sattels mit der 2D-Druckkarte des Prüfsattels, wenn die 2D-Druckkarte des maßgeschneiderten Sattels mit der 2D-Druckkarte des Prüfsattels übereinstimmt, Bestimmen, dass das maßgeschneiderte Sattelpolster angemessen ist, andernfalls Wiederholen aller Schritte des Verfahrens, bis das maßgeschneiderte Sattelpolster angemessen ist.

15. Verfahren nach einem der Ansprüche 2 bis 14, ferner umfassend das Montieren des maßgeschneiderten Sattelpolsters an die Sattelschale und optional das Abdecken mit einer Deckschicht, wie beispielsweise einer Lederschicht.

## Revendications

1. Procédé **(10)** de fabrication d'un **coussin de selle personnalisé (30)**, tel qu'un coussin de selle de bicyclette, le procédé comprenant :
a. l'application d'une **pâte modelable (14)** sur une **coque de selle (16)** pour former une **selle de test (18)**, la pâte modelable étant déformable sous le poids du cycliste ;
b. le montage de la selle de test sur une bicyclette et le fait de laisser la pâte modelable de la selle de test se déformer sous le poids et le mouvement d'un **cycliste (12)** roulant à bicyclette pendant une session de conduite, la session de conduite comprenant au moins une position de conduite active ;
c. après que la pâte modelable déformée a atteint un état géométriquement stable, le positionnement d'une pluralité de **capteurs de pression (20)** sur la selle de test et l'enregistrement des pressions provenant de la pluralité de capteurs de pression pendant une autre exécution de la session de conduite par le cycliste ;
d. l'obtention d'une **carte de pression 2D (22)** sur la pâte modelable déformée géométriquement stable en sélectionnant pour chaque capteur de pression la pression la plus élevée enregistrée pendant la session de conduite ;
e. l'obtention d'un **modèle 3D de coussin de selle (21)** de la pâte modelable déformée géométriquement stable ; et
f. l'association du modèle 3D de coussin de selle avec la carte de pression 2D pour obtenir un **maillage de pression 3D de coussin de selle (24)** destiné à servir de référence pour l'impression 3D du coussin de selle personnalisé, une densité du maillage de pression 3D de coussin de selle étant corrélée à la carte de pression 2D.

2. Procédé selon la revendication précédente, comprenant en outre l'impression 3D du coussin de selle personnalisé.

3. Procédé selon la revendication précédente, dans lequel la pâte modelable comprend une pluralité de couches de pâtes ayant des rigidités différentes.

4. Procédé selon la revendication précédente, dans lequel les couches de pâtes sont disposées les unes sur les autres par niveau de rigidité à partir d'une pâte ayant la rigidité la plus élevée disposée à proximité de la coque de selle.

5. Procédé selon l'une quelconque des revendications précédentes, dans lequel la pâte modelable est au moins l'une parmi une pâte minérale, thermoactivée et réutilisable.

6. Procédé selon l'une quelconque des revendications précédentes, dans lequel la pâte modelable est plastiquement déformable dans une plage de températures allant de la température ambiante à la température corporelle.

7. Procédé selon l'une quelconque des revendications précédentes, dans lequel le positionnement de la pluralité de capteurs de pression sur la selle de test comprend le positionnement sur la selle de test d'un tapis comprenant la pluralité de capteurs de pression.

8. Procédé selon l'une quelconque des revendications précédentes, dans lequel l'obtention du modèle 3D de coussin de selle comprend l'obtention du modèle 3D de coussin de selle par au moins l'un parmi le balayage, la radiographie et la photographie.

9. Procédé selon l'une quelconque des revendications précédentes, dans lequel l'association du modèle 3D de coussin de selle avec la carte de pression 2D comprend : la formation d'une carte de pression 3D de la carte de pression 2D et son association avec le modèle 3D de coussin de selle.

10. Procédé selon l'une quelconque des revendications précédentes, dans lequel la formation de la carte de pression 3D de la carte de pression 2D comprend l'application d'une dimension supplémentaire constante à la carte 2D.

11. Procédé selon l'une quelconque des revendications précédentes, dans lequel la session de conduite comprend deux positions de conduite actives ou plus dans lesquelles le cycliste pédale, et les deux positions de conduite actives ou plus étant au moins deux ou plus parmi : le cycliste avec les bras levés et le dos droit, le cycliste avec les bras baissés et le dos droit, le cycliste avec les mains sur le guidon de la bicyclette et le dos droit, le cycliste avec les mains sur le guidon tout en se penchant en avant vers le guidon.

12. Procédé selon l'une quelconque des revendications précédentes, dans lequel la pâte modelable peut être déformée, et facultativement durcie de manière réversible, une pluralité de fois.

13. Procédé selon l'une quelconque des revendications précédentes, dans lequel le maillage de pression 3D de coussin de selle est constitué d'alvéoles ouvertes.

14. Procédé selon l'une quelconque des revendications 2 à 13, comprenant en outre :
a. l'assemblage du coussin de selle personnalisé imprimé en 3D sur la coque de selle pour former une selle personnalisée et le montage de la selle personnalisée sur la bicyclette ;
b. le positionnement de la pluralité de capteurs de pression sur la selle personnalisée ;
c. l'enregistrement de la pression provenant de la pluralité de capteurs de pression pendant encore une autre exécution par le cycliste de la session de conduite ;
d. l'obtention d'une carte de pression 2D du coussin de selle personnalisé en sélectionnant pour chaque capteur de pression la pression la plus élevée enregistrée pendant la session de conduite ; et
e. la comparaison de la carte de pression 2D de la selle personnalisée avec la carte de pression 2D de la selle de test, si la carte de pression 2D de la selle personnalisée coïncide avec la carte de pression 2D de la selle de test, déterminer que le coussin de selle personnalisé est adéquat, sinon répéter toutes les étapes du procédé jusqu'à ce que le coussin de selle personnalisé soit adéquat.

15. Procédé selon l'une quelconque des revendications 2 à 14, comprenant en outre le montage du coussin de selle personnalisé sur la coque de selle, et facultativement son recouvrement par une couche de finition, telle qu'une couche de cuir.
